# EUROPEAN PATENT APPLICATION

(11) **EP 4 549 548 A1**
(43) Date of publication of application: **07.05.2025**
(21) Application number: 23830018.0
(22) Date of filing: 19.06.2023
(51) Int. Cl.: C12N 1/20, A61K 39/085, A61K 35/74, C12R 1/44

(54) **SYMBIOTIC BACTERIUM IN HUMANS FOR PROMOTING HAIR GROWTH AND USE THEREOF**

(30) Priority: 28.06.2022 CN 202210753866
(71) Applicant: Shanghai Microh Therapeutics, LLC, Shanghai 200120 (CN)
(72) Inventor: NIU, Chen, Shanghai 200120 (CN); XU, Tian, Shanghai 200120 (CN); TANG, Yuanyuan, Shanghai 200120 (CN); SUN, Yu, Shanghai 200120 (CN); MA, Zhenlin, Shanghai 200120 (CN); CHEN, Biao, Shanghai 200120 (CN)
(74) Representative: dompatent von Kreisler Selting Werner - Partnerschaft von Patent- und Rechtsanwälten mbB
(86) International application number: PCT/CN2023/101022
(87) International publication number: WO 2024/001838

(57) **Abstract**

Provided are a symbiotic bacterium in humans for promoting hair growth and use thereof. Specifically, provided is use of Staphylococcus hominis in treating alopecia or promoting hair growth, skin repair and regeneration and the like. Also provided is a composition for treating alopecia or promoting hair growth, including a pharmaceutical composition, a cosmetic composition, and the like.

## Description

### TECHNICAL FIELD

The present invention belongs to the field of biomedicine and cosmetics, and specifically, relates to symbiotic bacterium in humans for promoting hair growth and use thereof.

### BACKGROUND TECHNIQUE

Androgenic alopecia (AGA) is deemed as a hereditary alopecia disease caused by androgen dependence. This disease is more common in men. At present, the number of male alopecia in China is up to 130 million, and the male incidence rate is about 21.3%. For male androgenetic alopecia, most of the existing symptomatic drugs on the market are chemically synthesized drugs, which have unsatisfactory effects or significant side effects.

At present, there are no satisfactory, effective, and low side effect methods or drugs for treating alopecia or promoting hair growth in this field. Some medications for treating alopecia have various drawbacks. Minoxidil is administrated frequently and is prone to rebound after administration is stopped. Finasteride has significant side effects and a long administration period.

Therefore, there is an urgent need in the art to develop a new, safe and effective drug for treating alopecia or promoting hair growth.

### SUMMARY OF THE INVENTION

The purpose of the present invention is to provide a new safe and effective drug for treating alopecia or promoting hair growth, so as to treat symptoms such as androgenic alopecia.

In the first aspect of the present invention, it provides a symbiotic Staphylococcus, wherein the symbiotic Staphylococcus is *Staphylococcus hominis,* and the *Staphylococcus hominis* has a function of promoting hair growth.

In another preferred embodiment, the symbiotic Staphylococcus is *Staphylococcus hominis* ShC4, with a deposit number CCTCC NO. M 20211136.

In another preferred embodiment, the 16S rRNA gene sequence of the symbiotic Staphylococcus is as shown in SEQ ID NO.: 1 or has at least 99.5% identity.

In another preferred embodiment, the symbiotic Staphylococcus has an inflammatory regulatory effect.

In another preferred embodiment, the symbiotic Staphylococcus is isolated and extracted from human epidermis.

In the second aspect of the present invention, it provides a composition for promoting hair growth, which comprises: (a) a safe and effective amount of symbiotic Staphylococcus and/or its metabolites, wherein the symbiotic Staphylococcus is *Staphylococcus hominis;* and (b) a cosmetically or pharmaceutically acceptable carrier.

In another preferred embodiment, the composition further comprises an additional hair growth and/or hair care ingredient.

In another preferred embodiment, the symbiotic Staphylococcus comprises the symbiotic Staphylococcus of the first aspect of the present invention or its homologous symbiotic Staphylococcus.

In another preferred embodiment, the composition has a dosage form of (i) liquid preparation; (ii) solid preparation; or (iii) semi-solid preparation.

In another preferred embodiment, the composition is selected from the group consisting of a pharmaceutical composition, a cosmetic composition, and a combination thereof.

In another preferred embodiment, the liquid preparation is selected from the group consisting of: sterile normal saline, honey, and glycerin.

In another preferred embodiment, the dosage form of the composition is selected from the group consisting of: liquid preparation (spray, water, lotion), solid preparation (powder, tablet, powder injection, film, capsule), and semi-solid preparation (ointment, paste, emulsion, hydrogel).

In the third aspect of the present invention, it provides a use of the symbiotic Staphylococcus of the first aspect of the present invention or its homologous symbiotic Staphylococcus in preparation of a medicine, which is used for one or more purposes selected from the group consisting of: (a) treating alopecia or promoting hair growth; (b) enhancing hair follicular stemness; and (c) promoting skin repair and regeneration.

In the fourth aspect of the present invention, it provides a use of the symbiotic Staphylococcus of the first aspect of the present invention or its homologous symbiotic Staphylococcus in preparation of a medication or formulation, which is used for (i) promoting macrophage polarization towards M2 state; (ii) enhancing hair follicular stemness; and/or (iii) accelerating regeneration and repair of skin hair follicles.

In the fifth aspect of the present invention provides a method for preparing the composition of the second aspect of the present invention, comprising:
mixing the symbiotic Staphylococcus of the first aspect of the present invention or its homologous symbiotic Staphylococcus and/or its metabolites with a pharmaceutically acceptable carrier, thereby forming the composition of the second aspect of the present invention.

In another preferred embodiment, the composition is an external (or topical) preparation.

In the sixth aspect of the present invention, it provides a production method, comprising:
(a) cultivating the *Staphylococcus hominis* of the first aspect of the present invention under a suitable cultivation condition, thereby forming a culture product;
(b) optionally, isolating the *Staphylococcus hominis* and/or its metabolites from the culture product; and
(c) optionally, mixing the *Staphylococcus hominis* and/or its metabolites obtained from the previous step with a pharmaceutically acceptable carrier, thereby forming a composition.

In the seventh aspect of the present invention, it provides a personal care product, which comprises the symbiotic Staphylococcus of the first aspect of the present invention or its homologous symbiotic Staphylococcus.

In another preferred embodiment, the personal care product comprises hair growth product, hair care product, or skin care product.

In another preferred embodiment, the hair growth product comprises a hair growth product for preventing and/or treating alopecia.

In another preferred embodiment, the personal care product comprises liquid preparation, cream preparation, powder preparation and spray preparation, which is applied to an area for hair generation.

It should be understood that within the scope of the present invention, each technical feature of the present invention described above and each technical feature specifically described hereinafter (e.g., in the examples) may be combined with each other to form a new or preferred technical solution. Due to space limitations, they will not be repeated herein one by one.

### BRIEF DESCRIPTION OF DRAWINGS

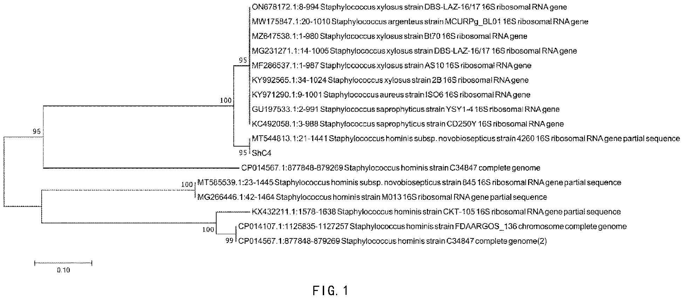

FIG. 1 shows the evolutionary relationship diagram of *Staphylococcus hominis* ShC4 (C4 marked in the following text and FIGs is ShC4) of the present invention, and the strain having the closest evolutionary relationship is another strain of *Staphylococcus hominis.*

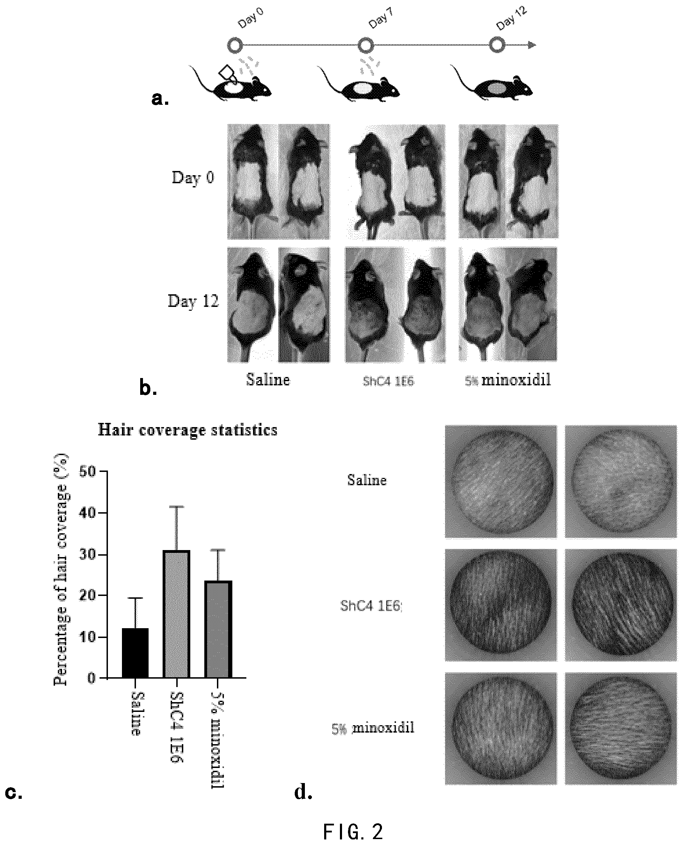

FIG. 2 shows the growth of mice hair under different administration conditions in animal experiments: a. animal experiment method: shaving the hair on the back of mice and removing the roots with alopecia cream (n=6 per group); the negative control is saline, and the positive control is 5% minoxidil; applying ShC4 bacteria to the back at a dose of 1 × 10⁶ cfu/mouse, and treating every 2 days; b. schematic diagram of hair growth on D12 (Day 12) in each group of mice; c. microscopic photography statistics of hair on D12 in each group of mice; d. hair microscopy photography, schematic diagram of hair coverage on D12.

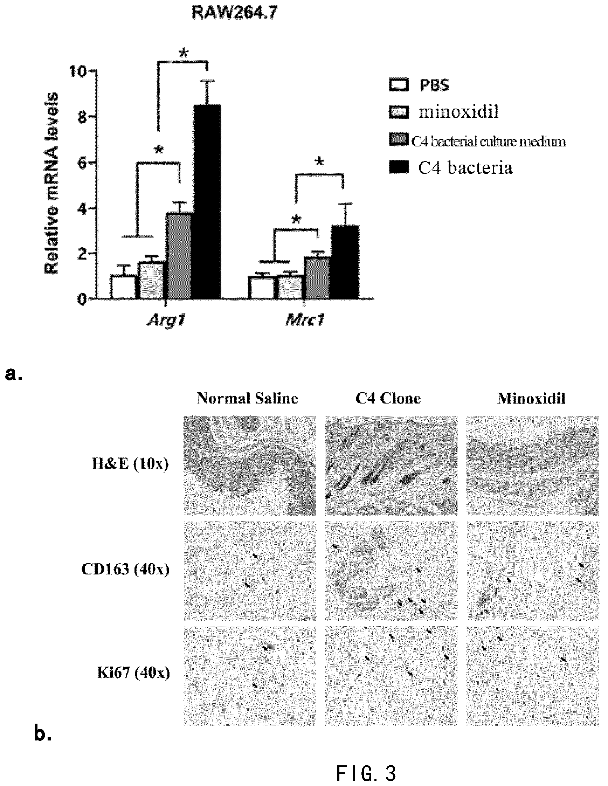

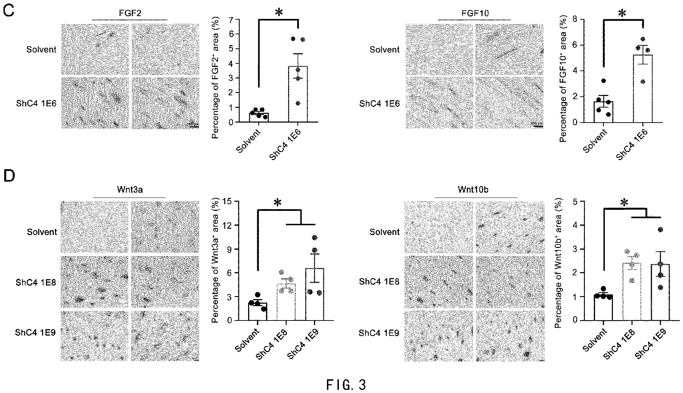

FIG. 3 shows that in cell and animal experiments, ShC4 bacteria (*Staphylococcus hominis*) promote macrophage M2 polarization and hair follicular stemness: a. it is shown that ShC4 bacteria induce M2 polarization of RAW264.7 macrophages derived from mice (marker genes Arg1, Mrc1); b. histochemical analysis IHC of dorsal skin samples of experimental mice (method shown in FIG. 2a.) (D10); control: 2% minoxidil, 100 µ L, once every 2 days, as indicated by the arrow, ShC4 bacteria induce M2 polarization of macrophages (marker CD163), enhance cell stemness (marker Ki67), and promote hair growth (H&E hair follicle staining), (C) immunohistochemical staining photos and quantitative results of FGF2 and FGF10 in the skin of ShC4 bacteria-treated mice; (D) immunohistochemical staining photos and quantitative results of Wnt3a and Wnt10b in the skin of ShC4 bacteria-treated mice; * p < 0.05.

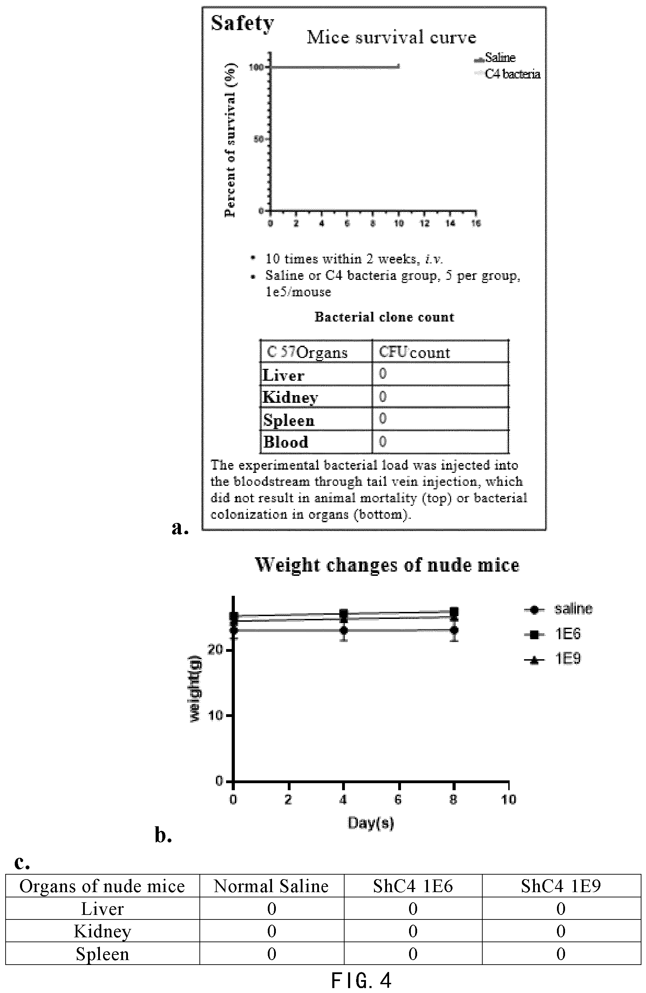

FIG. 4 shows the safety evaluation of ShC4 in wild and immunodeficient mice: a. wild mice were injected via tail vein (*i.v.*), b-c. nude mice were injected via tail vein (*i.v.*), (b) nude mice were weighed, and (c) mice were euthanized on Day 8. Liver, spleen, and kidney tissue were taken, homogenized and left to stand for 5 minutes. The supernatant was coated onto a plate and observed for colony growth at 37 °C for 24 hours. No colony growth was observed.

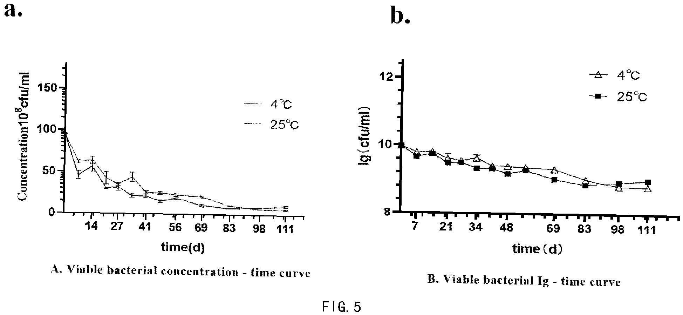

FIG. 5 shows the stability evaluation of the lyophilised preparation of ShC4 bacteria after exposure at 4 °C and 25 °C: a bacterial concentration count (cfu/mL), b. bacterial concentration decreased by less than 1 logarithm after 111 days.

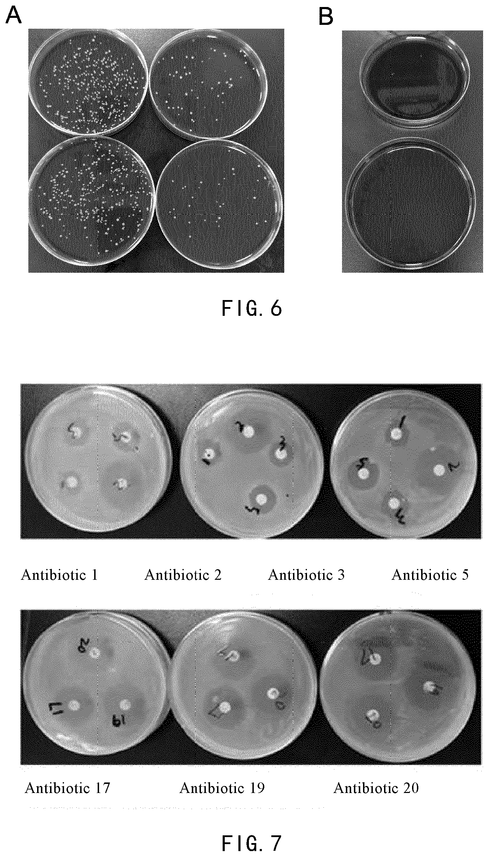

FIG. 6 shows the *in vitro* safety experiment of ShC4 strain: (A) hemolytic toxin detection experiment; (B) deoxyribonuclease detection experiment.
FIG. 7 shows the antibiotic susceptibility test of ShC4.

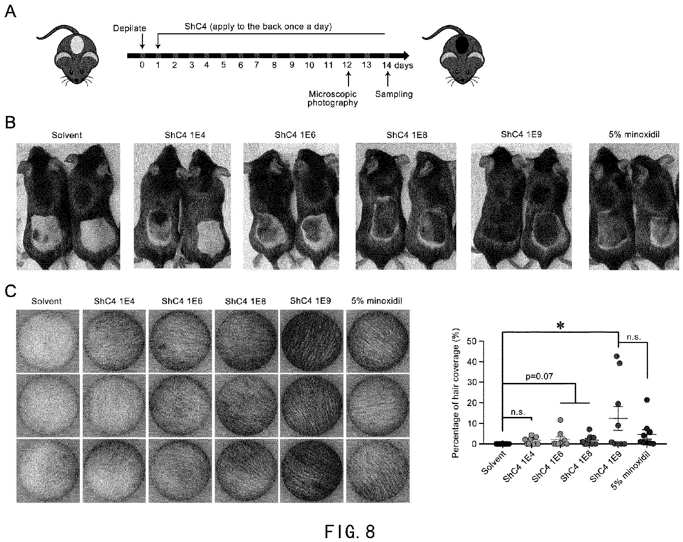

FIG. 8 shows the dose-effect experiment in a mouse depilatory alopecia model: (A) schematic diagram of animal experimental method; (B) schematic diagram of hair growth in each group of mice on Day 14; (C) microscopic photography and quantification results of hair in each group of mice on Day 12.

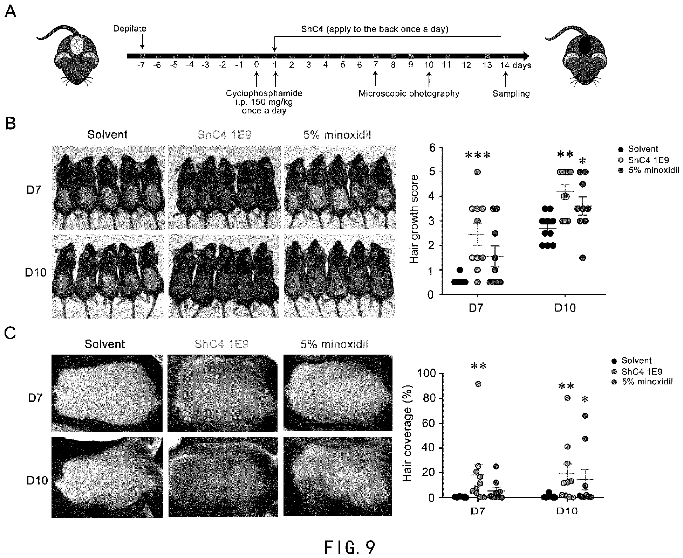

FIG. 9 shows the effectiveness experiment on the mouse cyclophosphamide alopecia model: (A) schematic diagram of animal experimental method; (B) schematic diagram and quantitative results of hair growth in each group of mice on Day 7 and Day 10; (C) microscopic photography and quantification results of the hair of mice in each group on Day 7 and Day 10.

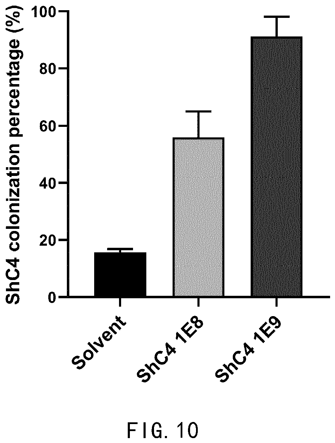

FIG. 10 shows the percentage of ShC4 in the mouse dorsal microbiota detected by 16S rRNA sequencing.

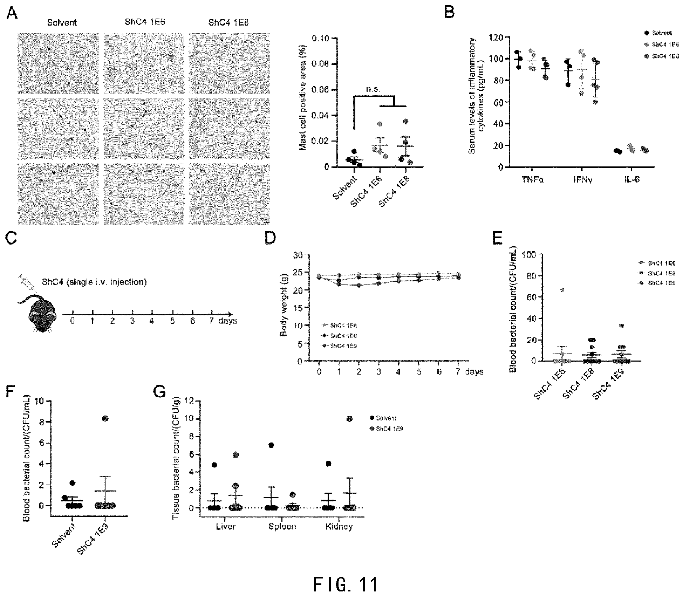

FIG. 11 shows the safety evaluation of ShC4 strain on C57BL/6N mice: (A) photos and quantitative results of toluidine blue staining of mouse skin; (B) the levels of serum inflammatory factors TNF α, IFN γ, and IL-6 in mice detected by ELISA; (C) schematic diagram of the experiment in which the mouse was injected with live ShC4 bacteria via the tail vein; (D) mouse weight curve; (E-F) bacterial concentration in mouse blood; (G) bacterial concentration in mouse liver, spleen, and kidney.

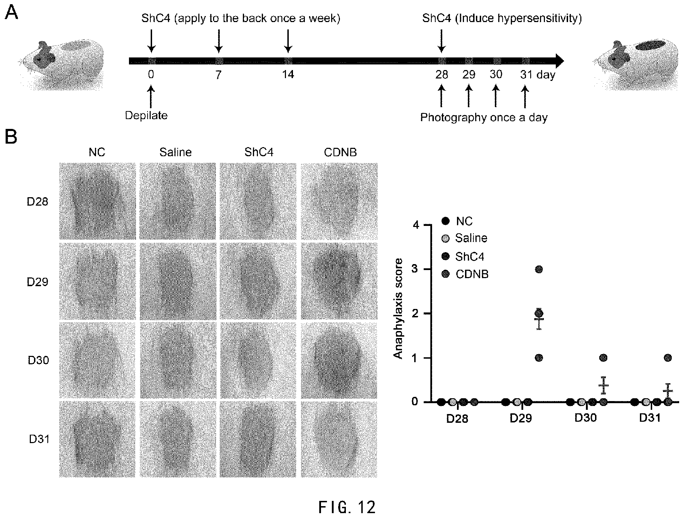

FIG. 12 shows the safety evaluation of ShC4 strain on guinea pig in active allergy test: (A) schematic diagram of the method of active allergy test in guinea pigs; (B) photographs of the back of guinea pigs at different times before and after active allergy provocation, as well as quantitative graphs of the scores.

### DETAILED DESCRIPTION

After extensive and intensive research and experimentation and through extensive screening, the inventors have unexpectedly obtained a symbiotic *Staphylococcus hominis* with functions such as promoting hair growth, namely *Staphylococcus hominis.* Cell experiments have shown that the *Staphylococcus hominis* of the present invention has the ability to treat alopecia and promote hair growth, skin repair, and regeneration. Topical application of an active composition containing *Staphylococcus hominis* to subjects can secrete various beneficial substances for the skin, promote macrophage polarization towards M2, enhance hair follicular stemness, accelerate skin hair follicle regeneration and repair, and effectively improve alopecia caused by excessive male hormone activity or secretion. On this basis, the present invention has been completed.

### Staphylococcus hominis and use thereof

As used herein, the terms "symbiotic Staphylococcus of the present invention", "*Staphylococcus hominis* of the present invention", and "*Staphylococcus hominis* of the present invention" are interchangeable and refer to the symbiotic Staphylococcus described in the first aspect of the present invention. The symbiotic Staphylococcus of the present invention is extracted from the human epidermis and has an inflammatory regulatory effect. In a preferred embodiment, the symbiotic Staphylococcus of the present invention is the following strain: *Staphylococcus hominis* ShC4, deposited under CCTCC NO. M 20211136.

The present invention provides the application of symbiotic Staphylococcus in treating alopecia and promoting hair growth. After back shaving, mice were treated with the *Staphylococcus hominis* ShC4 strain. The results showed that C57BL/6N male mice with shaved hair treated with the *Staphylococcus hominis ShC4* strain had significant hair growth compared to the control group. Therefore, the symbiotic Staphylococcus of the present invention has the ability to promote hair growth.

The symbiotic Staphylococcus of the present invention and its metabolites are used for improving, preventing, and/or treating various types of alopecia, including but not limited to: androgenic alopecia, drug (such as chemotherapy drug)-induced alopecia, radiation-induced alopecia, age-related alopecia, autoimmune disease induced alopecia, hormone induced alopecia, infection induced alopecia, or combinations thereof.

Representative drugs include (but are not limited to): drugs used for tumor treatment, such as cyclophosphamide, platinum compounds (cisplatin, carboplatin, etc.), paclitaxel and other chemotherapy drugs.

### Composition and its applications

The present invention also provides a composition, preferably a pharmaceutical composition. The composition includes an effective amount of *Staphylococcus hominis* of the invention. In a preferred embodiment, the composition also includes hair growth or hair care products selected from the following group consisting of: minoxidil, hair growth shampoo, scalp serum and the like.

In another preferred embodiment, the liquid formulation is selected from the group consisting of sterile saline, honey, and glycerol.

In another preferred embodiment, the dosage form of the composition is selected from the group consisting of: liquid preparation (spray, water, lotion), solid preparation (powder, tablet, powder injection, film, capsule), semi-solid preparation (ointment, paste, emulsion, hydrogel).

### Production method of Staphylococcus hominis

Usually, *Staphylococcus hominis* can be produced using conventional methods. In the present invention, a method is provided for large-scale production of *Staphylococcus hominis,* and specifically, it comprises the steps of:
(a) cultivating the *Staphylococcus hominis* under suitable cultivation conditions to obtain a culture product;
(b) optionally, isolating *Staphylococcus hominis* and/or its metabolites from the culture product; and
(c) optionally, mixing the *Staphylococcus hominis* and/or its metabolites obtained from the previous step with a food acceptable, cosmetically acceptable carrier or pharmaceutically acceptable excipient, and freeze-drying in vacuum to prepare a composition.

### Methods/products for inhibiting alopecia and/or promoting hair growth

In another preferred embodiment, the method comprises administering the pharmaceutical composition, cosmetic composition, or other combination of the present invention. The subject is human.

In another preferred embodiment, the method comprises: ingesting the pharmaceutical composition, cosmetic composition, or other combination of the present invention. The experimental subjects are animals, preferably rodents or rabbits.

The main advantages of the present invention include:
(a) The *Staphylococcus hominis* of the present invention belongs to the genus Staphylococcus and is a harmless symbiotic bacterium of human skin. It is widely distributed on the scalp, armpits, arms, and legs of the human body and can quickly and stably colonize the skin.
(b) The symbiotic Staphylococcus of the present invention is a skin symbiotic bacterium, which has a different therapeutic mechanism from that of the conventional alopecia compound drugs. Therefore, it can be used in combination with compound drugs with the same therapeutic effect.
(c) The symbiotic Staphylococcus of the present invention is screened from normal human skin tissue and belongs to a natural strain. It has not been subjected to genetic engineering or mutagenesis treatment, and has high safety. It will not have any side effects when it is applied topically.
(d) The formulation used in the present invention is easy to store and transport, convenient for medication, and easy to colonize on the skin.
(e) The ShC4 symbiotic bacteria of the present invention can enhance hair follicular stemness, thus having the effect of promoting hair regeneration.
(f) The ShC4 symbiotic bacteria of the present invention can effectively regulate the immune microenvironment, which is beneficial for hair regeneration and maintaining skin function.

The present invention will be further illustrated below with reference to the specific examples. It should be understood that these examples are only for illustrating the present invention and not intend to limit the scope of the present invention. Experimental methods not specified in the following examples are usually performed under conventional conditions, such as conditions described in Sambrook et al., Molecular Cloning: Laboratory Manual (New York: Cold Spring Harbor Laboratory Press, 1989), or as recommended by the manufacturer.

### Example 1: Screening and Identification of Staphylococcus hominis

### 1.1. Isolation of Staphylococcus hominis ShC4

The strain was isolated from the forehead area of healthy volunteers using monoclonal isolation. After screening and identification, it was unexpectedly discovered that a strain of *Staphylococcus hominis* had the function of promoting hair growth. The strain was named *Staphylococcus hominis* ShC4.

The human *Staphylococcus hominis* ShC4 was cultured using tryptone soy broth as the culture medium.

Culture medium formula: tryptone 17g/L, soy peptone 17g/L, glucose 2.5g/L, sodium chloride 5g/L, dipotassium dihydrogen phosphate 2.5g/L, sterile water 1000ml.

### 1.2. 16S rRNA gene identification

The 16S rRNA of the ShC4 strain of *Staphylococcus hominis* was sequenced and its gene sequence is shown in SEQ ID NO: 1.

tgcagtcgag cgaacagacg aggagcttgc tcctttgacg ttagcggcgg acgggtgagt 60
aacacgtagg taacctacct ataagactgg gataacttcg ggaaaccgga gctaataccg 120
gataatattt cgaaccgcat ggttcgatag tgaaagatgg ctttgctatc acttatagat 180
ggacctgcgc cgtattagct agttggtaag gtaacggctt accaaggcaa cgatacgtag 240
ccgacctgag agggtgatcg gccacactgg aactgagaca cggtccagac tcctacggga 300
ggcagcagta gggaatcttc cgcaatgggc gaaagcctga cggagcaacg ccgcgtgagt 360
gatgaaggtc ttcggatcgt aaaactctgt tattagggaa gaacaaacgt gtaagtaact 420
gtgcacgtct tgacggtacc taatcagaaa gccacggcta actacgtgcc agcagccgcg 480
gtaatacgta ggtggcaagc gttatccgga attattgggc gtaaagcgcg cgtaggcggt 540
tttttaagtc tgatgtgaaa gcccacggct caaccgtgga gggtcattgg aaactggaaa 600
acttgagtgc agaagaggaa agtggaattc catgtgtagc ggtgaaatgc gcagagatat 660
ggaggaacac cagtggcgaa ggcgactttc tggtctgtaa ctgacgctga tgtgcgaaag 720
cgtggggatc aaacaggatt agataccctg gtagtccacg ccgtaaacga tgagtgctaa 780
gtgttagggg gtttccgccc cttagtgctg cagctaacgc attaagcact ccgcctgggg 840
agtacgaccg caaggttgaa actcaaagga attgacgggg acccgcacaa gcggtggagc 900
atgtggttta attcgaagca acgcgaagaa ccttaccaaa tcttgacatc ctttgaccct 960
tctagagata gaagtttccc cttcggggga caaagtgaca ggtggtgcat ggttgtcgtc 1020
agctcgtgtc gtgagatgtt gggttaagtc ccgcaacgag cgcaaccctt aagcttagtt 1080
gccatcatta agttgggcac tctaagttga ctgccggtga caaaccggag gaaggtgggg 1140
atgacgtcaa atcatcatgc cccttatgat ttgggctaca cacgtgctac aatggacaat 1200
acaaagggca gcgaaaccgc gaggtcaagc aaatcccata aagttgttct cagttcggat 1260
tgtagtctgc aactcgacta catgaagctg gaatcgctag taatcgtaga tcagcatgct 1320
acggtgaata cgttcccggg tcttgtacac accgcccgtc acaccacgag agtttgtaac 1380
acccgaagcc ggtggagtaa ccatttggag ctagccgtcg a 1421
(SEQ ID No: 1)

The homology analysis results of *Staphylococcus hominis* ShC4 are shown in FIG. 1. The strain with the highest 16S rRNA homology with *Staphylococcus hominis* ShC4 is *Staphylococcus hominis* subsp. novelosepticus strain 4260. The homology is 95%.

### Example 2: The Promoting Effect of ShC4 Strain on Mouse Hair Growth

Animal source: C57BL/6N mice were purchased from Beijing Viton Lihua Laboratory Animal Technology Co. Male mice were selected for the experiment.

### 2.1 Experimental Method

The hair on the backs of the mice was shaved off, and the mice were randomly divided into three groups: normal saline control group, ShC4 1E6 group, and minoxidil group. Preparation of bacterial suspension: a monoclonal colony of the third generation of ShC4 was picked, and the culture method referred to Example 1. 30mL of the culture medium was centrifuged at 10,000 rpm for 10 minutes, and the supernatant was discarded. The precipitate was resuspended uniformly in 30 ml of sterile normal saline, and this process was repeated three times for standby use; the suspension was then diluted with sterile normal saline in gradients for standby use.

After modeling was completed, the drug was administered by dorsal application. On day 0, day 7, day 10, and day 12 after administration, the hair growth on the backs of the mice was observed and photographed using a hair microscope, and the hair coverage rate was calculated.

### 2.2 Experimental Results

As shown in FIG. 2, on the Day12 after modeling, the hair of the normal saline control group was relatively sparse, with a low coverage rate, while the minoxidil group had a promoting effect on mouse hair growth. The ShC4 treatment group showed a significant increase in hair coverage area (or ratio), suggesting that ShC4 treatment had a significant promoting effect on hair growth in depilated mice.

### Example 3: ShC4 induces M2 polarization of macrophages and enhances hair follicular stemness of skin tissue

### 3.1 Experimental Method

### 3.1.1 Detection of the ability of ShC4 to induce M2 of macrophage

Raw 264.7 cells (murine-derived macrophages) were stimulated with live ShC4 bacteria (MOI = 10) for 4 h, and stimulated with ShC4 bacterial metabolites (final concentration of 10 mg/mL) and minoxidil (final concentration of 50 µg/mL) for 24 h, respectively. The cell samples were collected, and total RNA was extracted, reverse-transcribed to cDNA, and detected by using RT-qPCR technology to obtain gene expression levels of the macrophage M2 type markers Arg1 and Mrc1.

### 3.1.2 The effect of ShC4 bacteria on hair follicular stemness

In order to explore the potential mechanism of ShC4 bacteria promoting hair growth in mice, the inventors collected dorsal skin samples of the mice from the solvent control group, ShC4 bacteria 1E9 CFU group, and 5% minoxidil positive control group in the dose effect experiment (refer to Example 2 for modeling methods), prepared paraffin sections, and performed H&E staining, as well as Ki67 and CD163 immunohistochemical staining.

### 3.1.3 Validation of the Effect of ShC4 Bacteria on Growth Factors

Research has shown that the activation of FGF family proteins, growth factors such as HGF and VEGF, and the TGF β signaling pathway can promote hair growth. To verify the hypothesis, paraffin sections of mouse skin tissue induced by ShC4 bacteria were used for immunohistochemical staining of FGF2 and FGF10, and quantitative analysis was performed.

### 3.1.4 Validation of the effect of ShC4 bacteria on the Wnt/β - catenin signaling pathway

Research has shown that activation of the Wnt/β - catenin signaling pathway promotes hair growth. To verify the hypothesis, paraffin sections of mouse skin tissue induced by ShC4 bacteria were used for immunohistochemical staining of Wnt3a and Wnt10b, and quantitative analysis was performed.

### 3.2 Experimental Results

The experimental results are shown in FIG. 3.

FIG. 3A shows that ShC4 bacteria and their culture medium supernatant can significantly upregulate the gene expression levels of Arg1 and Mrc1. This indicates that ShC4 bacteria and their metabolites can induce M2 polarization of macrophages, and the induction effect of ShC4 bacteria is stronger, while the positive drug minoxidil for treating alopecia does not have this function.

The staining results in FIG. 3B indicate that ShC4 bacteria can significantly enhance hair follicular stemness of mouse skin tissue.

The staining results in FIG. 3C show that compared with the control group, the protein expression levels of Fgf2 and Fgf10 are significantly upregulated in mouse skin tissue induced by ShC4 bacteria.

The staining results in FIG. 3D show that, compared with the control group, the protein expression levels of Wnt3a and Wnt10b are significantly upregulated in mouse skin tissue induced by ShC4 bacteria.

In summary, the above experimental results indicate that ShC4 bacteria can activate multiple signaling pathways, induce skin hair follicles to produce various growth factors, and promote hair growth.

### Example 4: Safety Evaluation Experiment of ShC4 in Mice

### 4.1 Experimental Method

Wild mice grouping: normal saline control group, ShC4 1E6 group. Ten wild mice were injected via the tail vein, wherein five mice were injected with normal saline as control, and five were injected with 100 µL of 1E6 ShC4 bacteria. Ten injections were performed within 2 weeks, and the mortality rate was calculated. After 2 weeks, the mice were dissected and the tissues were homogenized and coated on plates for CFU counting to detect bacterial residence.

Groups of nude mice: normal saline control group, ShC4 1E6 group, ShC4 1E9 group. Nude mice were injected with normal saline, 100 µL of 1E6 ShC4 bacteria and 100 µL of 1E9 ShC4 bacteria in the tail vein in a single injection. The body weight was observed and recorded within 8 days. After 8 days, the mice were dissected and the tissues were homogenized and coated on plates for CFU counting to detect bacterial residence.

### 4.2 Experimental Results

Wild mice were injected with ShC4 bacteria via tail vein 10 times within 2 weeks, and no animal death or bacterial residence in various organs were observed (FIG. 4a).

Within 8 days after administration, there was no weight loss in nude mice (FIG. 4b), and the plate coating results showed no ShC4 strain resided in each organ (FIG. 4c).

As shown in FIG. 4, the strain ShC4 of the present invention has good safety and is unable to colonise in animals (nude mice) with complete immunity or even underdeveloped thymus.

### Example 5: Stability Experiment

### 5.1 Experimental Method

The lyophilized formulation obtained by vacuum freeze-drying of ShC4 was placed at 4 °C and 25 °C, respectively. Samples are taken every 7 days to detect the concentration of viable bacteria in the samples, counted in cfu/mL.

### 5.1 Experimental results

The stability evaluation results of the lyophilized formulation of ShC4 bacteria are shown in FIG. 5. Both the bacterial concentration count (cfu/mL) (FIG. 5a) and the logarithmic viable bacteria (lg viable bacteria) (FIG. 5b) were maintained at a high level after exposure to 4°C and 25°C, and remained at the same logarithmic level (i.e., at least about 10^{8.8} cfu/ml) even at 111 days.

This indicates that *Staphylococcus hominis* ShC4 of the present invention has high stability.

### Example 6. In vitro safety assay of ShC4 strain

To verify the safety of the ShC4 strain, *in vitro* experiments were first conducted, in which haemolytic toxin, plasma coagulase and deoxyribonuclease were tested respectively.

Hemolytic toxins can damage platelets, disrupt lysosomes, and cause local ischemia and necrosis of the body. When *Staphylococcus aureus* invades the human body, plasma coagulase causes fibrin in the blood or plasma to deposit on the surface of the bacteria or coagulate, hindering phagocytosis by phagocytes. The easy localization of infection caused by *Staphylococcus aureus* is related to this enzyme, whereas deoxyribonuclease produced by *Staphylococcus aureus* can tolerate high temperatures and can be used as a basis for identifying *Staphylococcus aureus.*

### 6.1 Experimental Methods

### 6.1.1 Hemolytic toxin detection

Each batch of ShC4 strain product will be prepared from glycerol cryopreserved tubes from a working strain bank.

The glycerol cryopreserved tubes were thawed and inoculated into a modified soy protein broth medium (excluding animal protein) in a biosafety cabinet for cultivation. It was then cultured at 37 °C, with a shaking speed of 200 r/min, for 15h-18 hours. Afterwards, 0.8 mL of broth culture was added to a freeze-dried rabbit plasma vial, which was then gently shaken until the mixture was fully dissolved. The vial was incubated at 36±1°C, and observed every half hour for 6 hours. If coagulation (i.e., the formation of a clot when tilted or inverted) or a coagulation volume was greater than half of the original volume, it was deemed positive.

### 6.1.2 Plasma coagulase detection

Each batch of ShC4 strain product will be prepared from glycerol cryopreserved tubes from a working strain bank.

The glycerol cryopreserved tubes were thawed and inoculated into a modified soy protein broth medium (excluding animal protein) in a biosafety cabinet for cultivation. It was then cultured at 37 °C, with a shaking speed of 200 r/min, for 15-18 hours. Afterwards, 0.8 mL of broth culture was added to a freeze-dried rabbit plasma vial, which was then gently shaken until the mixture was fully dissolved. The vial was incubated at 36±1°C, and observed every half hour for 6 hours. If coagulation (i.e., the formation of a clot when tilted or inverted) or a coagulation volume was greater than half of the original volume, it was deemed positive.

### 6.1.3 Deoxyribonuclease detection

Each batch of ShC4 strain product will be prepared from glycerol cryopreserved tubes from a working strain bank.

The glycerol cryopreserved tubes were thawed and inoculated into a modified soy protein broth medium (excluding animal protein) in a biosafety cabinet for cultivation. It was then cultured at 37 °C, with a shaking speed of 200 r/min, for 15-18 hours. The broth culture was then subjected to a boiling water bath for 15 minutes. The treated broth culture was inoculated onto a toluidine blue-DNAse plate using an inoculating loop puncture, and incubated at 36±1 °C for 24 hours. If a faint pink color appeared around the puncture line, it was deemed positive.

### 6.2 Experimental Results

The experimental results are shown in FIG. 6. FIG. 6A shows that ShC4 did not form α, β, or γ hemolytic rings, indicating that it did not produce hemolysin. There was no obvious coagulation in the vial, so the ShC4 strain coagulase was negative. FIG. 6B shows that there was no color change around the periphery of the puncture of the ShC4 strain in experimental group A, while B was a blank plate, indicating that ShC4 strain was negative for heat-resistant nucleic acid.

In summary, strain ShC4 is negative for the hemolytic toxin, plasma coagulation enzyme, and deoxyribonuclease, indicating that the safety of the ShC4 strain is good.

### Example 7. ShC4 antibiotic sensitivity test

### 7.1 Experimental Method

The ShC4 strain was inoculated into a modified soy protein broth medium (without animal protein) and cultured under the conditions of 37°C, 200 r/min, for 15-18 hours. Then the bacterial concentration was assessed by measuring the OD₆₀₀ value. The bacteria were centrifuged at 10000 r/min, and the precipitated bacteria were washed twice with an equal volume of normal saline to remove the medium components. The bacterial density of the bacterial suspension was measured using OD₆₀₀ value.

The bacterial suspension was diluted to 0.5 MCF (with a viable bacterial concentration of 1.5x10⁸ cfu/ml), and 100 µL was evenly spread onto MH hydrolysed casein medium. Different types of antibiotic sensitivity tablets were placed on each plate, with three replicates for each type of antibiotic sensitivity tablet. The plates were inverted and incubated at 36±1 °C for 24 hours. The diameters of the inhibitory ring were measured using a ruler and recorded. The sensitivity of the ShC4 strain to various antibiotics was determined by referring to the NCCLS antibiotic sensitivity test standards.

### 7.2 Experimental results

The experimental results and data are shown in FIG. 7 and Table 1, respectively. ShC4 strain is sensitive to most antibiotics (e.g., benzoxicillin, piperacillin, tetracycline, minocycline, erythromycin, etc.) except for penicillin, ampicillin, and cotrimoxazole, to which ShC4 strain shows resistance.

**Table 1**

| NO. | Antibacterial drug name | Antibiotic content | Mean diameter (mm) | NLLCS standard Diameter of antibacterial ring (mm) | | | Sensitivity |
|---|---|---|---|---|---|---|---|
| | | | | R | I | S | |
| 1 | Penicillin (P) | 10U | 15.72 | ≤28 | - | ≥29 | R |
| 2 | Oxacillin (OX) | 10 µg | 29.4 | ≤17 | - | ≥18 | S |
| 3 | ampicillin | 10 µg | 16.69 | ≤28 | - | ≥29 | R |
| 5 | Piperacillin | 100 µg | 18.86 | ≤17 | - | ≥18 | S |
| 17 | tetracycline | 30 µg | 25.85 | ≤14 | 15-18 | ≥19 | S |
| 19 | minocycline | 30 µg | 29.33 | ≤14 | 15-18 | ≥19 | S |
| 20 | erythromycin | 15 µg | 23.96 | ≤13 | 14-22 | ≥23 | S |
| 27 | cotrimoxazole | 1.25 µg | 0 | | | | R |

### Example 8: Dose effect experiment on mouse depilatory alopecia model

### 8.1 Experimental subjects

Animal source: C57BL/6N mice were purchased from Shanghai Xipuer Bikai Experimental Animal Co., Ltd. Male mice aged 7 weeks ± 3 days were selected for the experiment.

### 8.2 Experimental Method

The hair on the backs of the mice was shaved off and the hair roots were removed using depilatory cream, and then the mice were randomly divided into six groups: solvent control group, ShC4 bacteria 1E4 CFU group, ShC4 bacteria 1E6 CFU group, ShC4 bacteria 1E8 CFU group, ShC4 bacteria 1E9 CFU group, and 5% minoxidil positive control group.

After the hair removal was completed, the ShC4 bacteria were administered by topical application on the backs of the mice (FIG. 8A).

The mice were photographed on the back on Day 14 of the experiment and microscopically photographed on Day 12 of the experiment, and the percentage of neonatal hair was quantified in response to the coverage of neonatal hair in the mice.

### 8.3 Experimental results

As shown in FIG. 8B, based on the comparison of photos taken on the 14th day of the experiment, it was found that as the dosage of ShC4 bacteria increased, the hair coverage on the back of mice in each group also showed a gradient increase. There was no significant difference in the ShC4 1E4 and 1E6 groups relative to the solvent control group, but the hair coverage of the ShC4 1E8 and 1E9 groups was significantly higher than that of the solvent control group, and not weaker than that of the 5% minoxidil positive control group.

As shown in FIG. 8C, the statistical results showed that there was no difference in hair coverage between the ShC4 1E4 group and the solvent control group. The hair coverage of the ShC4 1E6 and 1E8 groups showed an upward trend relative to the solvent control group, while the hair coverage of the ShC4 1E9 group showed a significant increase relative to the solvent control group, and there was no significant difference as compared to the 5% minoxidil positive control group. The average coverage was even higher than that of the 5% minoxidil positive control group.

In summary, the experiment showed that the ShC4 strain has a significant promoting effect on hair growth in depilated mice.

### Example 9. Effectiveness experiment of cyclophosphamide induced baldness model in mice

### 9.1 Experimental subjects

Animal source: C57BL/6N mice were purchased from Shanghai Xipuer Bikai Experimental Animal Co., Ltd. Male mice aged 7 weeks ± 3 days were selected for the experiment.

### 9.2 Experimental Method

The hair on the backs of the mice was shaved off and the hair roots were removed using depilatory cream, and then the mice were randomly divided into three groups: solvent control group (10 mice), ShC4 bacteria 1E9 CFU group (10 mice), and 5% minoxidil positive control group (9 mice).

The experimental process is shown in FIG. 9A. The formal experiment started from 7 days after hair removal, recorded as D0. Mice were intraperitoneally injected with 150 mg/kg cyclophosphamide for two consecutive days to induce baldness. On the day of the second injection of cyclophosphamide, ShC4 bacteria and minoxidil were administered by dorsal application, denoted as D1, for 14 consecutive days.

### 9.3 Experimental Results

As shown in FIG. 9B, mice were photographed on their backs on D7 and D10 of ShC4 bacteria application, respectively.

The comparison of scores showed that on D7, compared with the solvent control group, the hair coverage rate of the ShC4 1E9 group significantly increased, while there was no significant change in the 5% minoxidil group. Compared with the solvent control group, the 5% minoxidil group showed a significant increase in hair coverage on D10, while the increase in hair coverage in the ShC4 1E9 group was even more significant.

As shown in FIG. 9C, microscopic photographs were taken on mice coated with ShC4 bacteria on D7 and D10, respectively.

Through calculation and comparison, it was found that the results were consistent with the macroscopic photography results. On D7, as compared with the solvent control group, the hair coverage rate of the ShC4 1E9 group was significantly increased, while there was no significant change in the 5% minoxidil group. Compared with the solvent control group, the 5% minoxidil group showed a significant increase in hair coverage on D10, while the increase in hair coverage in the ShC4 1E9 group was even more significant.

In summary, the experiment showed that the ShC4 strain has a significant promoting effect on hair growth in cyclophosphamide induced balding mice.

### Example 10: Composition of Skin Microbiota after application of ShC4

### 10.1 Single application

C57BL/6N mice were divided into four groups: 0 h group, 0.5 h group, 8 h group, and 24 h group. Their backs were depilated (as in Example 8) to form a hair removal area of approximately 6 cm². A predetermined amount of ShC4 bacteria (1E9/mouse) were applied to the hair removal area. Skin samples were taken from the mice in the 0 h group before application, and samples were taken from the corresponding groups of mice in the application area at 0.5 h, 8 h, and 24 h after application for detection of skin microbiota.

The colonization of ShC4 on the back of mice was detected using16S rRNA sequencing.

The sequencing results showed that a single application of ShC4 bacteria resulted in a negative correlation between the number of Staphylococcus on the back of mice and the duration of administration. The proportion of ShC4 microbiota in the 0 h group before application was 0.8%, while after 0.5 hours of application, the proportion of ShC4 microbiota in the mouse back was 63.7% and increased significantly. After 8 hours of application of ShC4, the proportion of ShC4 in the mouse dorsal microbiota was 37.45%, significantly decreased compared to the 0.5h group, but still had a higher abundance. But after 24 hours of application of ShC4, the proportion of ShC4 in the mouse back microbiota was 3.2%, and the proportion of ShC4 significantly decreased, almost returning to the state before application, indicating that a single application of ShC4 did not stay in the skin for more than 24 hours.

### 10.2 Multiple applications

C57BL/6N mice were divided into three groups: control group, ShC4 1E8 group, and ShC4 1E9 group. Their backs were depilated (as in Example 8) to form a hair removal area of approximately 6 cm². A predetermined amount of ShC4 bacteria (1E8/mouse; or 1E9/mouse) were applied to the hair removal area daily for 14 days. At the end of the application, after 24 hours, the coated area was sampled for skin microbiota.

The colonization of ShC4 on the back of mice was detected using16S rRNA sequencing.

The sequencing results showed that the number of Staphylococcus on the back of mice was positively correlated with the amount of ShC4 bacteria administered. Compared to the control group, there was a significant increase in the colonization of Staphylococcus (mainly ShC4) on the back (approximately 6 cm²) of mice treated with ShC4 in the 1E8 and 1E9 groups (FIG. 10).

### Example 11. Animal safety experiment indicates that ShC4 induces no inflammatory response

### 11.1 Experimental subjects

Animal source: C57BL/6N mice were purchased from Shanghai Xipuer Bikai Experimental Animal Co., Ltd. Male mice aged 7 weeks ± 3 days were selected for the experiment.

### 11.2 Skin lesion application experiment

### 11.2.1 Experimental Method

C57BL/6N mice were randomly divided into three groups: solvent control group, ShC4 bacteria 1E6 CFU group, and ShC4 bacteria 1E8 CFU group, with 5 mice in each group. After the mice were depilated, 240-mesh sandpaper was used to scrape the back for the same number of times to create skin lesions, followed by the immediate application of the corresponding dose of ShC4 bacteria.

Samples were taken 3 days later, and no deaths of mice occurred during the experiment. The dorsal skin was fixed and processed into paraffin sections, which were then stained with toluidine blue to assess mast cell infiltration.

### 11.2.2 Experimental results

The staining and statistical results showed that there was no significant difference in the number of mast cells in the dermis of mice in the bacteria-coated group compared to the solvent group (FIG. 11A-B). In addition, mouse blood was centrifuged to obtain serum, and the levels of inflammatory factors were detected using an ELISA kit. The results showed that there was no significant difference in the levels of inflammatory factors TNF α, IFN γ, and IL-6 in the serum of mice in the bacteria-coated group compared to the solvent group. In summary, ShC4 bacteria have good safety and will not exacerbate inflammation in mice with skin lesions.

### 11.3 Experiment on tail vein injection of viable bacteria

### 11.3.1 Experimental Method

C57BL/6N mice were randomly divided into three groups: ShC4 1E6 CFU group, ShC4 1E8 CFU group, and ShC4 1E9 CFU group, with 10 mice in each group.

The mice were injected with 100 µL of ShC4 viable bacterial preparations containing different bacterial concentrations via tail vein on day 0, and observed continuously for 7 days with body weight measurement (FIG. 11C). No mouse deaths were found during the experiment, and the body weight of each group of mice remained stable without significant fluctuations (FIG. 11D).

### 11.3.2 Experimental results

50 µL of blood was taken from mice for plate coating, and it was found that a small amount of bacteria remained in the blood of each group of mice. However, there was no significant difference in the concentration of bacteria in the blood between the groups (FIG. 11E), indicating that even with high-dose injection of ShC4 bacteria, the immune system of mice could still suppressed the bacterial count at an extremely low level and ultimately eliminated bacteria.

In order to eliminate environmental bacterial contamination during the sampling process and compare the distribution of bacteria in various tissues and organs, the experiment was redesigned. C57BL/6N mice were randomly divided into two groups: solvent control group and ShC4 bacteria 1E9 CFU group, with 6 mice in each group.

On day 0, mice were injected with 100 µL of sterile water or ShC4 viable bacterial preparation containing 1E9 CFU via tail vein, and observed continuously for 7 days. After 7 days, 50 µL of blood was taken from mice for plate coating, and the liver, spleen, and kidneys were ground, homogenized, and coated. It was found that the number of bacteria in the blood, liver, spleen, and kidneys of mice injected with ShC4 bacteria via tail vein showed no significant difference as compared to the solvent control group, and most of them were zero (FIG. 11F-G). In some mice, including the solvent control group, a small amount of bacterial colonies appeared in their blood and tissues, which were considered to be caused by environmental pollution during subsequent operations, rather than residual ShC4 bacteria.

In summary, ShC4 bacteria have good safety and can be cleared by the immune system even if they are introduced into the blood, without affecting the health of mice.

### Example 12. Animal safety experiment indicates that ShC4 induces no skin allergy

### 12.1 Experimental subjects

Animal source: Guinea pig, from the Experimental Animal Center of the School of Pharmacy, Fudan University, strain: Dunkin Hartley; 3-month-old male guinea pigs were selected for the experiment.

### 12.2 Experimental method for active allergy of guinea pigs

Dunkin Hartley guinea pigs were randomly divided into four groups: negative control group (4 mice), matrix control group (4 mice), test sample group (7 mice), and positive control group (8 mice).

The guinea pigs were depilated on their backs, and the corresponding reagents were applied on D0, D7, and D14 after depilation. 200 µL of normal saline was applied to the matrix control group, 200 µL of 2E10 CFU ShC4 was applied to the test sample group, and 200 µL of 2,4-dinitrochlorobenzene (CDNB) was applied to the positive control group.

On D28, the back of guinea pigs was depilated again, and corresponding reagents were applied to each group again to stimulate allergies. Photos were taken before stimulation (D28), 24 hours after stimulation (D29), 48 hours after stimulation (D30), and 72 hours after stimulation (D31) for evaluation (FIG. 12A).

### 12.3 Experimental Results

Through comparisons of photos and scores, it was found that the allergy rate in the negative control group and the matrix control group was 0%, and the allergy rate in the positive control group reached 100%, while the allergy rate in the test sample group was also 0% (FIG. 12B). According to the evaluation criteria for sensitization intensity in the skin sensitization test outlined in the "Testing Methods for Chemicals", it can be concluded that ShC4 is a non-sensitizing substance to guinea pig skin.

In summary, ShC4 demonstrates good safety and does not cause any allergic reaction in guinea pigs.

### Strain deposit

The *Staphylococcus hominis* ShC4 of the present invention was deposited on September 6, 2021 at the China Center for Type Culture Collection (CCTCC) (Wuhan, China), under the deposit number CCTCC NO. M 20211136.

All references mentioned in the present application are incorporated by reference herein, as if each document were individually cited as a reference. In addition, it should be understood that after reading the above teaching content of the present invention, various changes or modifications may be made by those skilled in the art, and these equivalents also fall within the scope as defined by the appended claims of the present application.

| | | |
|---|---|---|
| 0-1 | **Form PCT/RO/134 Indications Relating to Deposited Microorganism(s) or Other Biological Material (PCT Rule 13bis)** | |
| 0-1-1 | Software Version | **i-system** |
| | | **Version 1.0.16 20230511 MT/FOP 20140331/0.20.5.21** |
| 0-2 | **International Application No.** | PCT/CN2023/101022 |
| 0-3 | **Applicant's or agent's file reference** | **P2023-0851-1PCWO** |
| | | |
| 1 | **The indications made below relate to the deposited microorganism(s) or other biological material referred to in the description on:** | |
| 1-1 | Page | **17** |
| 1-2 | Line | |
| 1-3 | **Identification of deposit** | |
| 1-3-1 | Name of depositary institution | **CCTCC China Center for Type Culture Collection** |
| 1-3-2 | Address of depositary institution | **College of Life Sciences, Wuhan University, Wuhan City, Hubei Province, China 430072** |
| 1-3-3 | Date of Deposit | **September 6th, 2021 (06.09.2021)** |
| 1-3-4 | Accession Number | **CCTCC NO.M 20211136** |
| 1-4 | **Supplementary indications** | |
| 1-5 | **Designated States for Which Indications are Made** | **All designations** |
| 1-6 | **The indications submitted separately.** These indications will be subsequently submitted to the International Bureau | |

| **Filled by the Receiving Office** | | |
|---|---|---|
| 0-4 | **This form was received with the international application:** (yes or no) | |
| 0-4-1 | Authorized Officer | |

| **Filled by the International Bureau** | | |
|---|---|---|
| 0-5 | **Date of Receipt of This Form by the International Bureau:** | |
| 0-5-1 | Authorized Officer | |

## Claims

1. A symbiotic Staphylococcus, wherein the symbiotic Staphylococcus is *Staphylococcus hominis,* and the *Staphylococcus hominis* has a function of promoting hair growth.

2. The symbiotic Staphylococcus of claim 1, wherein the symbiotic Staphylococcus is *Staphylococcus hominis* ShC4, with a deposit number CCTCC NO. M 20211136.

3. A composition for promoting hair growth, which comprises: (a) a safe and effective amount of symbiotic Staphylococcus and/or its metabolites, wherein the symbiotic Staphylococcus is *Staphylococcus hominis;* and a cosmetically or pharmaceutically acceptable carrier.

4. The composition of claim 3, which further comprises an additional hair growth and/or hair care ingredient.

5. The composition of claim 3, wherein the composition has a dosage form of (i) liquid preparation; (ii) solid preparation; or (iii) semi-solid preparation.

6. A use of the symbiotic Staphylococcus of claim 1 or its homologous symbiotic Staphylococcus in preparation of a medicine which is used for one or more purposes selected from the group consisting of: (a) treating alopecia or promoting hair growth; (b) enhancing hair follicular stemness; and (c) promoting skin repair and regeneration.

7. A use of the symbiotic Staphylococcus of claim 1 or its homologous symbiotic Staphylococcus in preparation of a medication or formulation which is used for (i) promoting macrophage polarization towards M2 state; (ii) enhancing hair follicular stemness; and/or (iii) accelerating regeneration and repair of skin hair follicles.

8. A method for preparing the composition of claim 3, comprising:
mixing the symbiotic Staphylococcus of claim 1 or its homologous symbiotic Staphylococcus and/or its metabolites with a pharmaceutically acceptable carrier, thereby forming the composition of claim 3.

9. A production method, comprising:
(a) cultivating the *Staphylococcus hominis* of claim 1 under a suitable cultivation condition, thereby forming a culture product;
(b) optionally, isolating *Staphylococcus hominis* and/or its metabolites from the culture product; and
(c) optionally, mixing the *Staphylococcus hominis* and/or its metabolites obtained from the previous step with a cosmetically or pharmaceutically acceptable carrier, thereby forming a composition.

10. A personal care product, which comprises the symbiotic Staphylococcus of claim 1 or its homologous symbiotic Staphylococcus.
